# EUROPÄISCHE PATENTSCHRIFT

(11) **EP 2 590 597 B1**
(45) Veröffentlichungstag und Bekanntmachung des Hinweises auf die Patenterteilung: **11.11.2015**
(21) Anmeldenummer: 11752460.3
(22) Anmeldetag: 10.06.2011
(51) Int. Cl.: A61F 2/46

(54) **VAKUUMZEMENTIERSYSTEM**
VACUUM CEMENTATION SYSTEM
SYSTÈME DE CIMENTATION SOUS VIDE

(30) Priorität: 07.07.2010 DE 102010026497
(43) Veröffentlichungstag der Anmeldung: 15.05.2013
(60) Teilanmeldung: 15187936.8
(73) Patentinhaber: Heraeus Medical GmbH, 61273 Wehrheim (DE)
(72) Erfinder: VOGT, Sebastian, 99092 Erfurt (DE); BUECHNER, Hubert, 90491 Nuernberg (DE); SCHNIEBER, Tim, 60439 Frankfurt (DE)
(74) Vertreter: Panten, Kirsten
(86) Internationale Anmeldenummer: PCT/EP2011/002867
(87) Internationale Veröffentlichungsnummer: WO 2012/003916

(56) Entgegenhaltungen:
- EP-A1- 1 741 413
- DE-A1- 19 532 015
- US-A1- 2003 155 381
- US-B1- 7 073 936

## Beschreibung

Die Erfindung betrifft ein Vakuumzementiersystem mit einem Behälteröffnungssystem zum Öffnen von Behältern.

Die Erfindung stellt Vakuumzementiersysteme zum Öffnen von Monomerampullen für Polymethylmethacrylat-Knochenzemente (PMMA-Knochenzemente), die mit Monomerflüssigkeit vorgefüllt sind, bereit, die als Full-Prepack-Vakuumzementiersysteme dem medizinischen Anwender zur Verfügung gestellt werden.

PMMA-Knochenzemente sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der PMMA-Knochenzemente ist seit dem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Bei dem Vermischen der Pulverkomponente mit der Monomerkomponente reagiert der Aktivator N,N-Dimethyl-p-Toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs bis dieser erstarrt.

Polymethylmethacrylat-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzementes verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird (Breusch SJ et al.: Der Stand der Zementiertechnik in Deutschland. Z Orthop. 1999, 137: 101-07). Im Vakuum gemischte Knochenzemente haben eine deutlich verringerte Porosität und zeigen daher verbesserte mechanische Eigenschaften. Es wurden eine Vielzahl von Vakuumzementiersystemen offen gelegt, von denen exemplarisch folgende genannt sind: US 5,624,184, US 4,671,263, US 4,973,168, US 5,100,241, WO 99/67015 A1, EP 1 020 167 A2, US 5,586,821, EP 1 016 452 A2, DE 36 40 279 A1, WO 94/26403 A1, EP 0 692 229 A1, EP 1 005 901 A2, US 5,344,232.

Vakuumzementiersysteme werden beim Mischen des PMMA-Knochenzements unter Vakuum gesetzt, um Lufteinschlüsse aus dem Zementteig zu entfernen. Es soll dadurch ein möglichst homogener, weitgehend blasenfreier Zementteig gebildet werden.

Eine Weiterentwicklung stellen Zementiersysteme dar, in denen sowohl das Zementpulver als auch die Monomerflüssigkeit bereits in separaten Kompartimenten der Mischsysteme verpackt sind und erst unmittelbar vor der Zementapplikation im Zementiersystem miteinander vermischt werden (US 5,997,544, EP 0 692 229 A1, US 6,709,149 B1). Problematisch ist bei diesen Systemen die Überführung der Monomerflüssigkeit in das Zementpulver und die vollständige Vermischung dieser beiden Komponenten, damit eine homogener Zementteig erhalten wird, der insbesondere keine Nester an nicht von der Monomerflüssigkeit benetzten Zementpulver enthalten darf. Bei dem gegenwärtig in Europa auf dem Markt befindlichen Full-Prepack-Mischsystem Optipac™ (Biomet Switzerland) wird durch einfache Röhren, die seitlich im unteren Teil der Kartusche angebracht sind, welche die Kartuschenwand durchstoßen, die Monomerflüssigkeit ungefähr in der Mitte des Zementpulvers durch Einwirkung von Vakuum aus Aluminiumverbundbeuteln in das Zementpulver eingesaugt. Aluminiumverbundbeutel werden zum Öffnen manuell gegen die Röhren bewegt, wobei die Röhren die Wandung der Beutel durchstechen.

Aluminiumverbundbeutel sind für die Verpackung und Lagerung von Monomerflüssigkeit erst seit wenigen Jahren bekannt. Sehr gute Erfahrungen hinsichtlich der Lagerfähigkeit von Monomerflüssigkeit liegen mit Glasampullen vor. Glasampullen werden seit Jahrzehnten für konventionelle Polymethylmethacrylat-Knochenzemente mit gutem Erfolg eingesetzt. Glasampullen haben neben der perfekten Dichtigkeit auch den Vorteil, dass sie in großen Stückzahlen zu niedrigen Preisen gefertigt werden können. Daher ist es sinnvoll, Glasampullen zur Verpackung und Lagerung von Monomerflüssigkeit in Prepack-Vakuumzementiersystemen einzusetzen.

In der DE 195 32 015 A1 ist eine Vorrichtung zum Mischen und Ausbringen von Mehrkomponentenprodukten beschrieben. Dabei wird eine Vorrichtung zum Öffnen von Ampullen vorgeschlagen, die darauf beruht, dass an der Außenseite der Zementkartusche eine Lagerbuchse ausgebildet ist, um die sich drehbar ein Ampullenhalter bewegen kann. Der Kopf der Ampulle befindet sich im Inneren der Lagerbuchse. Bei der Drehung des Ampullenhalters um die Lagerbuchse wird der Ampullenkopf vom Ampullenkörper abgescheert. Dann kann die Flüssigkeit aus der Ampulle in die Kartusche durch eine Öffnung in der Kartuschenwand überführt werden.

In der WO 97/18031 A1 wird eine Vorrichtung vorgeschlagen, bei der eine Ampulle am Boden durchstoßen wird und die Monomerflüssigkeit danach durch den hohlen Mischstab in die Zementkartusche fließen kann.

Ein System zum Öffnen von Ampullen bei Zementiersystemen wird in der EP 1 031 333 A1 offen gelegt. Bei diesem System wird durch eine manuelle Bewegung des Mischstabes gegen eine keilförmige Vorrichtung im Kartuschenkopf der Ampullenkopf schräg gegen die Ampullenachse bewegt, wobei der Ampullenkopf vom Ampullenkörper abgescheert wird. Problematisch sind hierbei der relativ komplexe Aufbau der Öffnungsvorrichtung und die Gefahr des Verklemmens der keilförmigen Vorrichtung.

In der WO 2010/012114 A1 ist eine Vorrichtung zum manuellen Öffnen von Ampullen beschrieben. Dabei handelt es sich analog zur DE 195 32 015 A1 um einen Drehmechanismus zur Abscheerung des Ampullenkopfes. Der einzige Unterschied zur DE 195 32 015 A1 besteht darin, dass eine Drehbuchse gegen den Ampullenhalter bewegt wird und nicht wie bei der DE 195 32 015 A1 der Ampullenhalter gegen die Drehbuchse.

Es kann als zusammenfassend festgestellt werden, dass alle vorgenannten Prepack-Vakuumzementiersystem manuell zu öffnende Monomerflüssigkeitsbehälter enthalten. Vorteilhafter wäre es, wenn bei dem Anlegen des Vakuums an das Zementiersystem eine zwangsweise automatische Öffnung der Monomerflüssigkeitsbehälter erfolgen würde. Dadurch wäre die Handhabung der Vakuumzementiersysteme für den medizinischen Anwender deutlich vereinfacht und sicherer.

US 2003/155381 A1 offenbart ein System und Verfahren zur Mischung von Knochenzement, wobei eine Monomerampulle in einer Ampullenbrecheinrichtung eingesetzt wird und über Vakuum gegen ein Hindernis gezogen wird, derart, dass der Ampullenkopf abgebrochen wird. Anschließend kann die Flüssigkeit aus der Ampulle herausgezogen werden.

Der Erfindung liegt die Aufgabe zu Grunde, ein Verfahren und eine Vorrichtung zu entwickeln, bei dem Monomerflüssigkeitsbehälter ohne manuelle Betätigung automatisch geöffnet werden und damit der Monomertransfer aus einem Monomerbehälter zu einem Zementbehälter automatisch ausgelöst werden kann.

Diese Aufgabe wird durch ein Vakuumzementiersystem mit mindestens einer Zementpulverkartusche und einem Behälteröffnungssystem gelöst, wobei das Behälteröffnungssystem wenigstens einen Behälter für zumindest eine Flüssigkeit und/oder zumindest ein Pulver, eine Öffnungsvorrichtung und eine Auslassöffnung zum Anlegen eines Unterdrucks umfasst, wobei bei Anlegen eines Unterdrucks die Öffnungsvorrichtung relativ zu zumindest einem Teil des Behälters beweglich ist, und das Vakuumzementiersystem ein Mittel zur Verbindung der Zementpulverkartusche mit dem Behälter umfasst, wobei das für die Herstellung eines blasenarmen Zementteigs benötigte Vakuum zusätzlich zum automatischen Öffnen des Behälters genutzt wird. Dabei umfasst das Behältersystem einen beweglich gelagerten Kolben, der durch den äußeren Druck relativ zu dem Behälter und auch relativ zu dem gesamten Behälteröffnungssystem bewegt wird und derart in dem Behälteröffnungssystem verschiebbar ist, dass der oder die Behälter durch die Bewegung des Kolbens geöffnet werden können, und der eine gabelförmige Führung zum Führen eines aufzubrechenden Teils des Behälters umfasst.

Der Behälter ist vorteilhaft in einer Kammer angeordnet und die Auslassöffnung an die Kammer angeschlossen.

Dabei wird durch die Bewegung des Kolbens zumindest ein Teil des Behälters abgebrochen, so dass der Kolben als Öffnungsvorrichtung wirkt.

Auch kann vorgesehen sein, dass der oder die Behälter eine Flüssigkeit, insbesondere eine Monomerflüssigkeit, und/oder ein Pulver, insbesondere ein Zementpulver, beinhalten, wobei der Inhalt nach dem Öffnen aus dem Behälter unter Einwirkung des Unterdrucks, insbesondere eines Vakuums, austritt und anschließend mit einem Pulver, insbesondere einem Zementpulver, oder einer Flüssigkeit, insbesondere einer Monomerflüssigkeit, unter Einwirkung des Unterdrucks, insbesondere eines Vakuums, durchmischt wird.

Dabei kann vorgesehen sein, dass das Gemisch, vorzugsweise mit Hilfe eines Überdrucks, aus einem Applikator herausgedrückt wird oder herausläuft.

Ein Behälteröffnungssystem, das zum Durchführen eines solchen Verfahrens geeignet ist, umfasst wenigstens einen Behälter für zumindest eine Flüssigkeit und/oder zumindest ein Pulver, eine Öffnungsvorrichtung und eine Auslassöffnung zum Anlegen eines Unterdrucks, wobei bei Anlegen eines Unterdrucks die Öffnungsvorrichtung relativ zu zumindest einem Teil des Behälters beweglich ist.

Dabei ist vorgesehen sein, dass das Behälteröffnungssystem einen beweglich gelagerten, insbesondere zylindrischen Kolben umfasst.

Die Erfindung sieht vor, dass der bewegliche Kolben eine gabelförmige Führung zum Führen eines aufzubrechenden Teils des Behälters umfasst.

Es kann auch vorgesehen sein, dass der Behälter eine Flüssigkeit umfasst, die eine Monomerkomponente, vorzugsweise ein polymerisierbares Monomer, wie zum Beispiel Methylmethacrylat, und einen darin gelösten Aktivator, enthält.

Bei dem Behälter handelt es sich vorzugsweise um eine Ampulle, insbesondere einen Monomerflüssigkeitsbehälter.

Dabei kann vorgesehen sein, dass die Zementpulverkartusche mindestens ein Mischorgan, insbesondere einen Förderkolben umfasst.

Es kann auch vorgesehen sein, dass ein statischer Mischer an einer Applikationsöffnung des Vakuumzementiersystems angeordnet ist.

Es kann ferner vorgesehen sein, dass die Zementpulverkartusche ein Pulver umfassend ein oder mehrere Polymere, insbesondere auf der Basis von Methylmethacrylat und Comonomeren, beinhaltet.

Unter einem mittelbaren Öffnen eines Behälters durch die Druckdifferenz ist zu verstehen, dass ein bewegliches Mittel, wie zum Beispiel ein Kolben, durch den äußeren Druck relativ zu dem Behälter und auch relativ zu dem gesamten Behälteröffnungssystem bewegt wird und diesen dadurch mechanisch öffnet, also beispielsweise durch Aufbrechen, Aufschneiden oder Einstechen eines Teils des Behälters beziehungsweise einer Behälterwand. Dazu wird das Mittel zum Öffnen des Behälters beweglich im Behälteröffnungssystem gelagert. Der Differenzdruck wirkt also auf das Mittel zur Öffnung, was in einer Bewegung des Mittels resultiert.

Ein unmittelbares Öffnen ist dann gegeben, wenn ein Behälter oder ein Teil eines Behälters durch die Druckdifferenz auf eine statische Öffnungsvorrichtung gedrückt wird. Der Differenzdruck wirkt hierbei also direkt und unmittelbar auf den beweglichen Behälter, so dass sich der Behälter gegen das Behälteröffnungssystem bewegt. Der Behälter ist dazu beweglich im Behälteröffnungssystem gelagert. Der Behälter wird dann auf eine Öffnungsvorrichtung, die mit dem Behälteröffnungssystem verbunden ist, geschoben beziehungsweise gedrückt und durch Aufbrechen, Aufschneiden oder Einstechen eines Teils des Behälters beziehungsweise einer Behälterwand mechanisch geöffnet.

Eine mittelbare Öffnung ist auch dann gegeben, wenn sich sowohl der Behälter als auch die Öffnungsvorrichtung bewegen, also beide beweglich im Behälteröffnungssystem gelagert sind.

Die Erfindung basiert auf der überraschenden Erkenntnis, dass ein Vakuum bei Vakuumzementiersystemen neben der Entfernung von Lufteinschlüssen aus dem Zementteig auch zum Öffnen von Behältern, die beispielsweise ein Monomer beinhalten, nutzbar gemacht werden kann. Somit wird das bei Vakuumzementiersystemen ohnehin für die Herstellung eines blasenarmen Zementteigs benötigte Vakuum zusätzlich auch zum automatischen Öffnen von Behältern, beispielsweise Monomerflüssigkeitsbehältern, genutzt. Durch die Erzeugung eines Unterdrucks im Zementiersystem wird die entstehende Druckdifferenz zur Umgebung zum Antrieb eines automatischen, mechanischen Öffnungssystems verwendet. Der Unterdruck bewirkt eine Relativbewegung einer Öffnungsvorrichtung, wie beispielsweise eines Kolbens, gegen den Behälter, wobei durch die Relativbewegung eine mechanische Krafteinwirkung auf den Behälter erfolgt. Durch diese Krafteinwirkung wird der Behälter geöffnet und dessen Inhalt, zum Beispiel ein Monomer, das zur Bildung von Knochenzement erforderlich ist, freigesetzt.

Unter dem Begriff Behälter im Sinne der Erfindung werden vorzugsweise Glasampullen, Kunststoffampullen, Aluminiumampullen und Kunststoffverbundbeutel, einschließlich Aluminiumverbundbeutel, verstanden.

Im Folgenden werden Ausführungsbeispiele der Erfindung anhand von schematisch dargestellten Zeichnungen erläutert. Dabei zeigen:
- Figur 1:: eine schematische Querschnittansicht in Längsrichtung eines erfindungsgemäßen Behälteröffnungssystems mit geschlossenem Behälter,
- Figur 2:: Eine schematische Aufsicht auf einen Kolben mit gabelförmiger Führung als Öffnungsvorrichtung für ein erfindungsgemäßes Vakuumzementiersystem,
- Figur 3:: Eine schematische Vorderansicht des Kolbens nach Figur 2,
- Figur 4:: Eine schematische perspektivische Ansicht des Kolbens nach Figur 2 und
- Figur 5:: Eine schematische Querschnittansicht eines erfindungsgemäßen Vakuumzementiersystems.

Beispiele für erfindungsgemäße Vakuumzementiersysteme sind in den folgenden Figurenbeschreibungen erläutert.

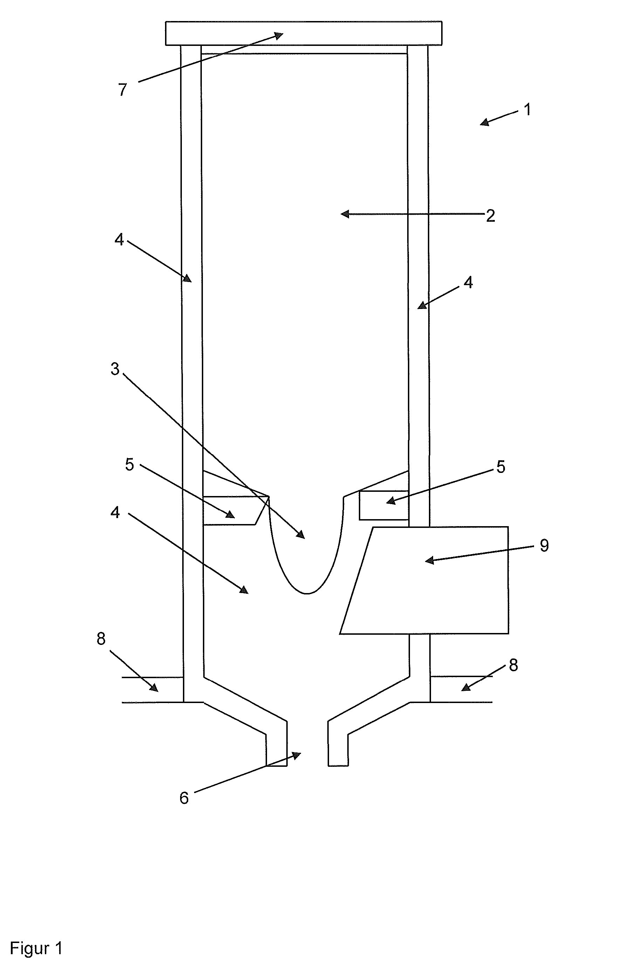

Figur 1 zeigt eine schematisch dargestellte Querschnittansicht eines Behälteröffnungssystems (1), in dem ein Behälter (2) in Form einer Ampulle angeordnet ist. Der Behälter (2) umfasst einen Behälterkopf (3) und gemäß Figur 1 einen darüber befindlichen Behälterkörper. Zwischen dem Behälterkopf (3) und dem Behälterkörper befindet sich eine Brechzone.

Der Behälter ist in einer Kammer (4) des Behälteröffnungssystems (1) angeordnet und durch zwei Stutzen (5) positioniert. Unterhalb des Behälterkopfs (3) ist eine Auslassöffnung (6) in der Kammer (4) vorgesehen. Die Oberseite der Kammer (4) ist durch einen Deckel (7) verschlossen. Der Deckel (7) ist durch ein Gewinde oder einen anderen Verschlussmechanismus mit der Kammer (4) verbunden. Das Behälteröffnungssystem (1) kann in einer anderen Vorrichtung, die hier durch die offenen Wände (8) dargestellt ist, eingebaut oder integriert sein.

Im Bereich des Behälterkopfs (3) ist eine zylindrische Öffnung in den Wänden der Kammer (4) vorgesehen, in der ein zylindrischer Kolben (9) entlang seiner Symmetrieachse beweglich angeordnet ist.

Die Wände der Kammer (4) und der Deckel (7) sind druckstabil, erhalten also ihre Form auch dann, wenn im Inneren der Kammer (4) ein Vakuum erzeugt wird. Der Kolben (9) steckt in gasdichter Presspassung in der Öffnung der Wände der Kammer (4).

Das Verfahren kann beispielsweise wie folgt durchgeführt werden: Zunächst wird an der Auslassöffnung (6) ein Unterdruck angelegt. Dadurch bildet sich auch im Inneren der Kammer (4) ein Unterdruck aus. Die Druckdifferenz zwischen dem Inneren der Kammer (4) und der äußeren Umgebung der Kammer (4) führt dazu, dass auf der nach außen weisenden Fläche des Kolbens (9) ein größerer Druck und damit eine größere Kraft wirkt als auf der nach innen weisenden Fläche des Kolbens (9). Der Kolben (9) erfährt daher eine resultierende Kraft, die ihn ins Innere der Kammer (4) treibt. Sobald die Haftreibung des in Presspassung sitzenden Kolbens (9) durch die resultierende Kraft überwunden ist, bewegt sich der Kolben (9) ins Innere der Kammer (4). Schließlich wird der Kolben (9) auf den Behälterkopf (3) treffen und diesen abscheren. Hierdurch wird der Behälter (2) geöffnet. Der Inhalt des Behälters (2) fließt aus diesem heraus und durch die Auslassöffnung (6) hindurch aus dem Behälteröffnungssystem (1) ab.

Da bei neuartigen Vakuumzementiersystemen ohnehin ein Vakuum erzeugt wird, um beispielsweise eine Monomerflüssigkeit mit einem Knochenzement unter Vakuum zu mischen, kann dieses Vakuum auch gleichzeitig dazu genutzt werden, den steril abgeschlossenen Behälter (2), in dem sich die Monomerflüssigkeit und/oder das Knochenzementpulver befindet, zu öffnen. Geeignete und geläufige Behälter (2) für die Monomerflüssigkeit beziehungsweise auch den Knochenzement sind beispielsweise Glasampullen.

Der Haftreibungswiderstand des Kolbens (9) in der Öffnung der Kammer (4) und/oder die Stabilität der Brechzone des Behälters (2) beziehungsweise der Querschnitt des Kolbens (9) können dabei so eingestellt werden, dass sich der Behälter (2) beziehungsweise die Glasampulle erst dann öffnet, wenn der Unterdruck in der Kammer (4) dazu ausreicht, ein ausreichend blasenfreies Gemisch aus Monomerflüssigkeit und Knochenzement zu erzeugen.

Die Öffnungsvorrichtung stellt in diesem Ausführungsbeispiel der beweglich gelagerte Kolben (9) dar.

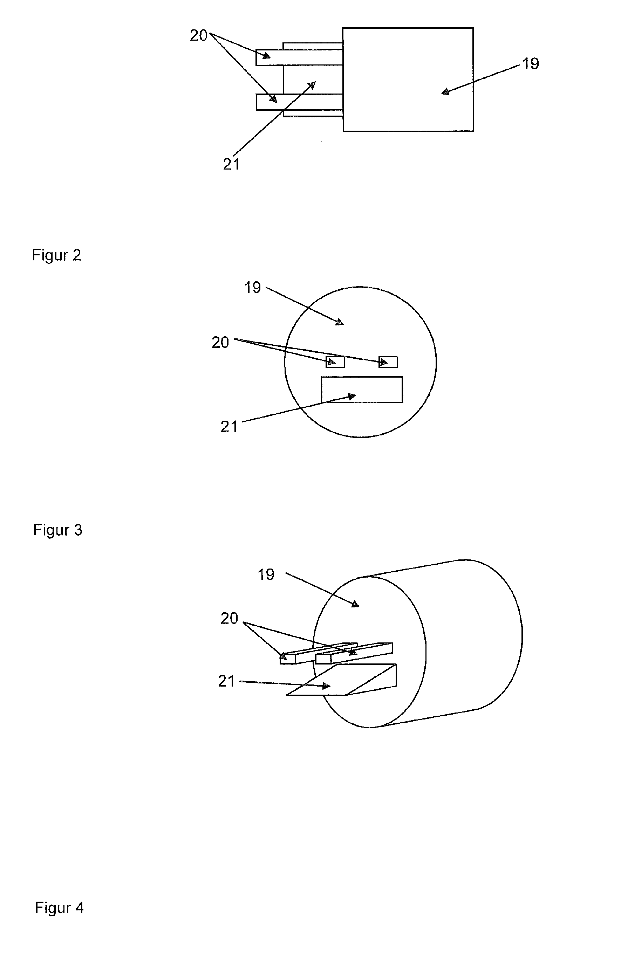

Die Figuren 2, 3 und 4 zeigen in verschiedenen Ansichten schematische Darstellungen eines erfindungsgemäß gestalteten Kolbens (19) für erfindungsgemäße Behälteröffnungssysteme (nicht gezeigt). Der Kolben (19), der als Öffnungsvorrichtung für Behälter fungieren soll, umfasst eine gabelförmige Führung bestehend aus zwei Stegen (20) und umfasst des Weiteren eine abgeschrägte vorstehende Platte (21). Die gabelförmige Führung (20) soll die Bewegung des Kolbens (19) relativ zu einem Behälterkopf definieren. Ein hierfür geeigneter Behälter hat eine Verjüngung zwischen dem Behälterkopf und dem Behälterkörper, in den die gabelförmige Führung (20) greifen kann.

Mit der Platte (21) kann entweder ein Behälterkopf abgebrochen beziehungsweise abgeschert werden oder die Kante der Platte (21) ist so scharf, dass sie in der Lage ist, die Wände eines Behälters zu durchschneiden. Die gabelförmige Führung (20) und die Platte (21) dienen dazu, den Öffnungsvorgang reproduzierbarer zu machen und genauer durchführen zu können. Der Zwischenraum zwischen den beiden Stegen (20) kann sich zum Kolben (19) hin verjüngen, um dadurch ein Abtrennen eines Behälterkopfs bei der Bewegung des Kolbens (19) zu ermöglichen. Die Platte (21) ist dann nicht mehr notwendig.

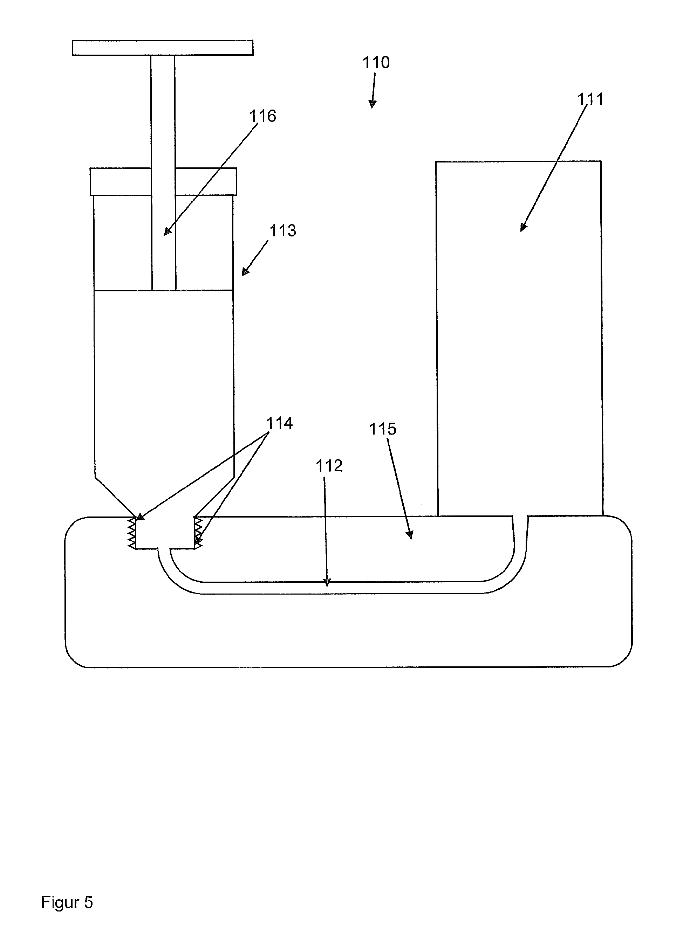

Figur 5 zeigt ein Vakuumzementiersystem (110) umfassend ein Behälteröffnungssystem (111) umfassend eine mit einer Flüssigkeit gefüllte Glasampulle als Behälter (nicht gezeigt), die über ein Verbindungsmittel (112), wie beispielsweise eine Leitung, mit einer Zementpulverkartusche (113) verbunden ist. Die Zementpulverkartusche (113) ist über Befestigungsmittel (114), wie beispielsweise einem Gewinde, mit einem Fußteil (115) des Vakuumzementiersystems (110) verbunden. Die Zementpulverkartusche (113) umfasst einen Förderkolben (116), mit dem der Inhalt der Kartusche (113) aus dem Vakuumzementiersystem (110) ausgetrieben, beziehungsweise der fertig gemischte Zement appliziert werden kann.

Mit Hilfe eines Vakuums wird zunächst die Glasampulle im Behälteröffnungssystem (111), wie in den anderen Figuren gezeigt, geöffnet. Die Flüssigkeit aus dem Behälter wird durch das Vakuum über das Verbindungsmittel (112) in die Zementpulverkartusche (113) gesaugt und vermischt sich dort mit dem Zement. Mit Hilfe des Förderkolbens (116) kann das Gemisch appliziert werden. Ein statischer Mischer (nicht gezeigt) kann in der Applikationsöffnung (nicht gezeigt) des Vakuumzementiersystems (110) vorgesehen sein, um eine stärkere Durchmischung der Flüssigkeit mit dem Zement zu erreichen.

Besonders vorteilhaft sind das erfindungsgemäße Verfahren und das erfindungsgemäße Vakuumzementiersystem (111) für PMMA-Knochenzemente geeignet, wobei als Flüssigkeit eine Monomerkomponente (die beispielsweise Methylmethacrylat und einen Aktivator enthält) und als Pulverkomponente ein aus einem oder mehreren Polymeren bestehendes Knochenzementpulver (beispielsweise auf der Basis von Methylmethacrylat und Comonomeren) verwendet wird, beziehungsweise in dem Behälter und der Zementpulverkartusche (113) enthalten sind.

Für alle Ausführungsbeispiele gilt, dass unterhalb der Auslassöffnung (6) ein Sieb und/oder ein Filter (nicht gezeigt) angeordnet sein kann, mit dem Splitter beziehungsweise Reste des Behälters (2), die beim Abbrechen des Behälterkopfs (3) entstehen können, zurückgehalten werden.

Der Behälter (2) ist vorzugweise eine Ampulle, insbesondere eine Glasampulle.

Beim Öffnen von Ampullen in Vakuumzementiersystemen kann durch Einwirkung eines Vakuums mit einem absoluten Druck von größer gleich 70 mbar auf mindestens einen Kolben, der Kolben relativ zu einem oder mehreren Monomerflüssigkeitsbehälter(n) verschoben werden, und dadurch der oder die Monomerflüssigkeitsbehälter geöffnet werden, wobei die Öffnung durch Aufbrechen erfolgt.

Erfindungsgemäß kann ferner vorgesehen sein, dass der Kolben (9) senkrecht zur Ampullenachse angeordnet ist und an der Kolbenseite, die in Richtung des Inneren der Kammer (4) gewandt ist, senkrecht oder schräg zur Wandfläche der Kammer (4,) ausgebildet ist, wobei die Kolbenseite eine glatte Oberfläche oder eine gefurchte Oberfläche besitzen kann. Bei Beaufschlagung mit Vakuum bewegt sich der Kolben (9, 19) in Richtung Ampullenkopf (3) und schert diesen gegen den Ampullenkörper ab.

Erfindungsgemäß ist eine gabelförmige Führung (20) an der Innenseite des Kolbens (9, 19) angebracht, wobei der Abstand der Stege der Führung (20) gleich groß oder größer als der Durchmesser der Ampulle (2,) in der Brechzone ist. Die Führung (20) kann zwischen dem Ampullenkopf (3) und der Ampullenschulter die Ampulle (2132) gegen senkrechte Bewegungen entlang der Ampullenachse fixieren. Dadurch kann die Ampulle (2) bei einem Verschieben des Kolbens (9, 19) auf den Ampullenkopf (33) nicht ausweichen. Es wird ein einfacherer Bruch der Ampulle (2, , 132) erreicht.

### Bezugszeichenliste

- 1, 111: Behälteröffnungssystem
- 2: Behälter
- 3: Behälterkopf
- 4: Kammer
- 5: Stutzen
- 6: Auslassöffnung
- 7: Deckel
- 8: offene Wände
- 9: Kolben
- 20: gabelförmige Führung / Steg
- 21: abgeschrägte Platte
- 110: Vakuumzementiersystem
- 112: Verbindungsmittel
- 113: Zementpulverkartusche
- 114: Befestigungsmittel
- 115: Fußteil
- 116: Förderkolben

## Patentansprüche

1. Vakuumzementiersystem umfassend mindestens eine Zementpulverkartusche (113) und ein Behälteröffnungssystem, wobei das Behälteröffnungssystem wenigstens einen Behälter (2) für zumindest eine Flüssigkeit und/oder zumindest ein Pulver, eine Öffnungsvorrichtung (9) und eine Auslassöffnung (6) zum Anlegen eines Unterdrucks umfasst, wobei bei Anlegen eines Unterdrucks die Öffnungsvorrichtung (9) relativ zu zumindest einem Teil des Behälters (2) beweglich ist, und das Vakuumzementiersystem ein Mittel (112) zur Verbindung der Zementpulverkartusche (113) mit dem Behälter (2) umfasst, wobei das für die Herstellung eines blasenarmen Zementteigs benötigte Vakuum zusätzlich zum automatischen Öffnen der Behälter (2) genutzt wird, wobei das Behälteröffnungssystem einen beweglich gelagerten Kolben (9, 19) umfasst, der durch den äußeren Druck relativ zu dem Behälter und auch relativ zu dem übrigen Behälteröffnungssystem bewegt wird und derart in dem Behälteröffnungssystem verschiebbar ist, und wobei der oder die Behälter durch die Bewegung des Kolbens geöffnet werden können, **dadurch gekennzeichnet, dass** der beweglich gelagerte Kolben eine gabelförmige Führung (20) zum Führen eines aufzubrechenden Teils (3) des Behälters (2) umfasst.

2. Vakuumzementiersystem nach Anspruch 1, **dadurch gekennzeichnet, dass** der Behälter (2) in einer Kammer (4) angeordnet ist und die Auslassöffnung (6) an die Kammer angeschlossen ist.

3. Vakuumzementiersystem nach einem der vorhergehenden Ansprüche, **dadurch gekennzeichnet, dass** die Zementpulverkartusche (113) mindestens ein Mischorgan (116), insbesondere einen Förderkolben (116) umfasst.

4. Vakuumzementiersystem nach einem der vorhergehenden Ansprüche, **dadurch gekennzeichnet, dass** ein statischer Mischer an einer Applikationsöffnung des Vakuumzementiersystems (110) angeordnet ist.

## Claims

1. Vacuum cementing system, comprising at least one cement powder cartridge (113) and a container opening system, whereby the container opening system comprises at least one container (2) for at least one liquid and/or at least one powder, an opening device (9), and an outlet opening (6) for application of a negative pressure, whereby the opening device (9) is mobile relative to at least a part of the container (2) when a negative pressure is being applied, and the vacuum cementing system comprises a means (112) for connecting the cement powder cartridge (113) to the container (2), whereby the vacuum required for producing a bubble-depleted cement dough is additionally used to automatically open the container (2), **characterised in that** the container system comprises a plunger (9, 19) that is supported such as to be mobile and is moved, through the external pressure, relative to the container and also relative to the entire container opening system and can be shifted in the container opening system in such manner that the container or containers can be opened through the motion of the plunger, and which comprises a fork-shaped guidance (20) for guiding a part (3) of the container (2) that is to be broken open.

2. Vacuum cementing system according to claim 1, **characterised in that** the container (2) is arranged in a chamber (4) and the outlet opening (6) is connected to the chamber.

3. Vacuum cementing system according to any one of the preceding claims, **characterised in that** the cement powder cartridge (113) comprises at least one mixing organ (116), in particular a feed plunger (116).

4. Vacuum cementing system according to any one of the preceding claims, **characterised in that** a static mixer is arranged on one application opening of the vacuum cementing system (110).

## Revendications

1. Système de cimentation sous vide, comportant au moins une cartouche de poudre de ciment (113) et un système d'ouverture de récipient, le système d'ouverture de récipient comportant au moins un récipient (2) pour au moins un liquide et/ou au moins une poudre, un dispositif d'ouverture (9) et un orifice d'évacuation (6) pour appliquer une dépression, le dispositif d'ouverture (9) pouvant se déplacer, par l'application d'une dépression, par rapport à au moins une partie du récipient (2), et dont le système de cimentation sous vide comporte un moyen (112) pour la liaison de la cartouche de poudre de ciment (113) avec le récipient (2), le vide nécessaire pour l'élaboration d'une pâte de ciment pauvre en bulles étant en plus utilisé pour l'ouverture automatique des récipients (2), **caractérisé en ce, que** le système de récipients comporte un piston (9, 19) monté de manière mobile, qui, par la pression extérieure, soit mis en mouvement par rapport au récipient et aussi par rapport à l'ensemble du système d'ouverture de récipient et qui soit déplaçable dans le système d'ouverture de récipient de telle sorte, que le ou les récipients puissent être ouverts par le mouvement du piston, et qui comporte un guidage (20) en forme de fourche pour guider une partie (3) à ouvrir du récipient (2).

2. Système de cimentation sous vide selon la revendication 1, **caractérisé en ce, que** le récipient (2) soit agencé dans une chambre (4) et que l'orifice d'évacuation (6) soit raccordé à la chambre.

3. Système de cimentation sous vide selon l'une des revendications précédentes, **caractérisé en ce, que** la cartouche de poudre de ciment (113) comporte au moins un organe de mélange (116), en particulier un piston de refoulement (116).

4. Système de cimentation sous vide selon l'une des revendications précédentes, **caractérisé en ce, qu'**un mélangeur statique soit agencé à une ouverture d'application du système de cimentation sous vide (110).
